# EUROPEAN PATENT APPLICATION

(11) **EP 2 140 826 A1**
(43) Date of publication of application: **06.01.2010**
(21) Application number: 08011789.8
(22) Date of filing: 30.06.2008
(51) Int. Cl.: A61B 19/00, A61B 19/02, G01B 15/04, G01N 23/04

(54) **Identification of a product in a package**

(71) Applicant: Stryker Trauma GmbH, 24232 Schönkirchen/Kiel (DE)
(72) Inventor: Carstens, Henning, 24211 Preetz (DE); Amiralai, Saleh, 24241 Schierensee (DE)
(74) Representative: Maiwald Patentanwalts GmbH

(57) **Abstract**

A method and device for identification of a product in a package using an X-ray line scanner, which allows a fast, secure and more automated packaging and monitoring process, in particular for medical and surgical products, for example, implants, which underlie a high requirement with respect to a secure handling.

## Description

### Field of the Invention

The present invention relates to a method for identification of a product in a package and a device for identification of a product in a package, and in particular to a method and a device for identification of a product in a package allowing an automated monitoring of the products in a package.

### Background of the Invention

Surgical products like, for example, bone nails, bone plates or bone screws generally are packed after a manufacturing process in order to, for example, sterile covering of the surgical product. For this purpose, the medical or surgical products will be covered by, for example, a plastic bag and sealed, and thereafter heated or otherwise sterilizes by, for example, UV rays or gamma radiation in order to obtain a sterile product. However, it is a large effort to monitor the packing process as well as the monitoring of the manufacturing parameters of the surgical products. The product parameters and the correspondence thereof with a target parameter has to be checked, and further, it is to be checked whether the correct product is correctly packed and labelled before purchasing.

### Summary of the Invention

It would be desirable to provide a device and method for identification of a product in a package, which allows an automated packaging and monitoring process.

The invention provides a method and device for identification of a product in a package, a corresponding programme element and a computer readable medium according to the subject matter of the independent claims. Further embodiments are incorporated in the dependent claims.

It should be noted that the following described exemplary embodiments of the invention apply also for the method, the device, the programme element and the computer readable medium.

According to an exemplary embodiment of the invention, there is provided a method for identification of a product in a package, the method comprising imaging the product, determining an outer contour of the product based on the imaging of the product in a package, determining a geometry of the product based on the outer contour of the product, determining parameters of the product based on the determined geometry of the product, comparing determined parameters of the product with respective target parameters of the product, and evaluating the product as a conform product or as a non-conform product.

Thus, it is possible to identify the product by determining an outer contour of the product, which allows to check whether, for example, the correct product is provided with the correct package and/or correct labelling. Further, the determined geometry of the product allows to determine the parameters of the product by, for example, an algorithm, so that several parameters can be identified, which are characteristic parameters for the product. Those characteristic parameters can be compared with respective target parameters of the product to check out whether the manufacturing of the product has been correct, or whether there are any unacceptable deviations of the characteristic parameters rendering the product untreatable for use, in particular for medical purposes. Thus, the identified and checked product can be evaluated as a conform product to be delivered or as a non-conform product, which may be re-crafted or sorted out. Thus, it is possible to provide an automated process for evaluating whether the correct product is present in the package, whether it corresponds with a respective labelling and/or it corresponds with corresponding documents belonging to the respective product.

According to an exemplary embodiment of the invention, the imaging of the product is an X-ray imaging of the product.

Thus, it is possible to provide an imaging of the product irrespective of the colour and opaqueness of the package. In other words, the imaging of the product may take place also in cases where the product is covered by a non transparent plastic bag, or a carton case.

According to an exemplary embodiment of the invention, determining the outer contour of the product comprises determining a greyscale image of the product based on the imaging of the product and separating the product from an environment of the product by applying a pre-determined threshold to the greyscale image.

Thus, the outer contour may be provided irrespective of the environment, so that the environment can be eliminated. The contour of the product my serve as an identifier which allows to identify the product out of a pre-determined group of possible products. Thus, it can be easily determined whether the packed product corresponds to the intended product, which can be, for example, obtained by comparing the identified product with a content of a labelling. Applying a pre-determined threshold to the greyscale image allows a fast processing and a fast identification of the product.

According to an exemplary embodiment of the invention, determining a geometry of the product comprises determining a greyscale image of the product based on the outer contour of the product and determining the geometry of the product based on a plurality of greyscales of the greyscale image.

Thus, not only the contour of the product can be determined, but also the whole geometry, e.g. the curvature, the dimensions and other parameters, so that also further parameters may be determined as a base for evaluating whether the product is a conform product or a non-conform product. In particular, the plurality of greyscales allows a kind of three dimensional information, which allows to also include parameters which cannot be obtained only from the contour of the product. Thus, also parameters with respect to the thread or different materials may be determined. It should be noted that it is also possible to use a kind of coloured image, which requires an imaging process based on different wavelengths of radiation. It should be noted that those images do not mandatorily have to be images in the visible range of light, but may also be obtained by radiation having a plurality of wavelengths and being outside the visible range of light. Coloured image may be considered as an image which is obtained by a plurality of wavelengths of the imaging radiation.

According to an exemplary embodiment of the invention, determining of parameters of the product comprises determining a parameter out of a group, the group consisting of dimension, material, shape, radius, angle, diameter, hole diameters and threads.

Thus, relevant parameters may be determined with respect to the quality monitoring of the product for maintaining the product parameters within allowable tolerances.

According to an exemplary embodiment of the invention, comparing determined parameters of the product with respective target parameters of the product comprises comparing the determined parameters with a look-up table.

Thus, it is possible to implement a fast evaluating process and to allow to update a look up table depending on the presently distributed products.

According to an exemplary embodiment of the invention, the method further comprises reading a label on a package, identifying a label content, allocating to the label content corresponding target parameters and comparing the determined parameters of the product with allocated target parameters.

Thus, it is possible to check whether the correct product is packed in a correct package and labelled with a correct label, respectively. This in particular allows a more integrated automisation process allowing not only to check out the product parameters, but also the monitoring of the correct destination of the manufactured, packed and labelled product.

According to an exemplary embodiment of the invention, a method further comprises sorting the product based on evaluating the product as a conform product or as a non-conform product.

Thus, it is possible to sort the product into a group which is suitable for use, in particular suitable for medical use of the product, which can be considered as a conform product, and in a group which includes the non-conform products. The latter may further be divided in a sub group including products which are suitable for being re-crafted in order to meet the respective requirements after re-crafting and in a sub group including products which have to be finally sorted out.

According to an exemplary embodiment of the invention the method further comprises providing a tag on the package based on evaluating the product as a conform product as a non-conform product and sealing the package together with the tag.

Thus, it is possible to provide, for example, a tag which indicates a correct sterilisation process within the sealing of the outer package which avoids a manipulation, in particular with respect to the medically important property of sterilisation of the product. In other words, the tag indicates a correct sterilisation process but is provided within a closed sealing. The tag may further be provided with an identifier which indicates a damage of the sealing of the package, e.g. by a oxygen sensitive layer.

According to an exemplary embodiment of the invention, there is provided a programme element, which, when being executed by a processor, is adapted to carry out the inventive method, as described above.

According to an exemplary embodiment of the invention, there is provided a computer readable medium having stored the inventive programme element, as described above.

According to an exemplary embodiment of the invention, there is provided a device for identification of a product in a package, wherein the device comprises an imaging device being adapted for imaging the product, a contour determining device being adapted for determining an outer contour of the product based on the imaging of the product in a package, a geometry determining device being adapted for determining a geometry of the product based on the outer contour of the product, a parameter determining device being adapted for determining parameters of the product based on the determined geometry of the product, a comparing device being adapted for comparing determined parameters of the product, with respective target parameters of the product, and an evaluating device being adapted for evaluating the product as a conform product or as a non-conform product.

According to an exemplary embodiment of the invention, the imaging device is an X-ray line scanner.

Thus, it is possible to obtain an image of the product in a package, which image has a high resolution and being suitable for being analysed with respect to the contour and the geometry, and thus with respect to the parameters of the product.

It should be noted that the above features may also be combined. The combination of the above features may also lead to synergetic effects, even if not explicitly described in detail.

These and other aspects of the present invention will become apparent from and elucidated with reference to the embodiments described hereinafter.

### Brief Description of the Drawings

Exemplary embodiments of the invention will be described in the following with reference to the following drawings.
Fig. 1 illustrates a general schematic flow-chart of an embodiment of the inventive method.
Fig. 2 illustrates the determination of a contour of a product in a package.
Fig. 3 illustrates determining a geometry of the product in a package.
Fig. 4 illustrates determining parameters of the product based on the contour and the geometry of the product.
Fig. 5 illustrates a product in a package.
Fig. 6 illustrates an image of the product in a package, in particular an image obtained by a line scanner.
Fig. 7 illustrates determining and evaluating a geometry, in particular a hole position in the product.
Fig.8 illustrates a device according to an exemplary embodiment of the invention.

### Detailed Description of Exemplary Embodiments

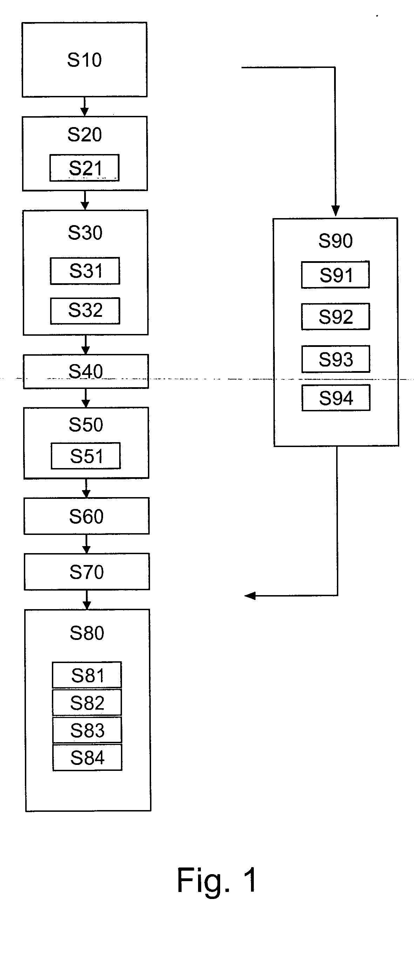

Fig. 1 illustrates a schematic overview over the inventive method according to an exemplary embodiment of the invention. According to the inventive method, the product in a package will be imaged S10. The imaging may take place by, for example, X-ray imaging or any other imaging process. An X-ray imaging process is of particular interest if the package is not transparent, so that with an X-ray imaging also completely covered and opaque packages can be imaged and the respective product be evaluated. In step S20 the outer contour of the product will be determined based on the imaging of the product in a package. Determining an outer contour of the product may, for example, include a separating process S21 to separate the product from, for example, the environment of the product on the imaged product. In step S30 the geometry of the product is determined based on the outer contour of the product, wherein the step of determining a geometry of the product may also include determining a greyscale image of the product S31 and determining the geometry of the product based on a plurality of greyscales as step S32. Afterwards, in step S40, determining parameters of the product takes place based on the determined geometry of the product. Then, in step S50, the determined parameters are compared with respective target parameters of the product. This step may also include comparing the determined parameters with a look-up table S51 in order to implement a fast processing. In step S60, the product will be evaluated as a conform product or as a non-conform product.

According to a further exemplary embodiment of the invention, the product can be sorted based on evaluating the product as a conform product or as a non-conform product in step S70, and can further be provided with an identifier in step S80, wherein this step may include providing a tag to the package S81 based on evaluating the product as a conform product or as a non-conform product and also sealing the package together with a tag in step S82. Further, the package may also be provided with a label S83 and may be sealed afterwards S84. In a possibly parallel process it can be determined in step S90, whether the product is correctly packed with the correct labelling and the correct destination. In particular, this may include reading a label on a package S91, identifying a label content S92, allocating to the label content corresponding target parameters S93 and comparing the determined parameters of the product with allocated target parameters S94.

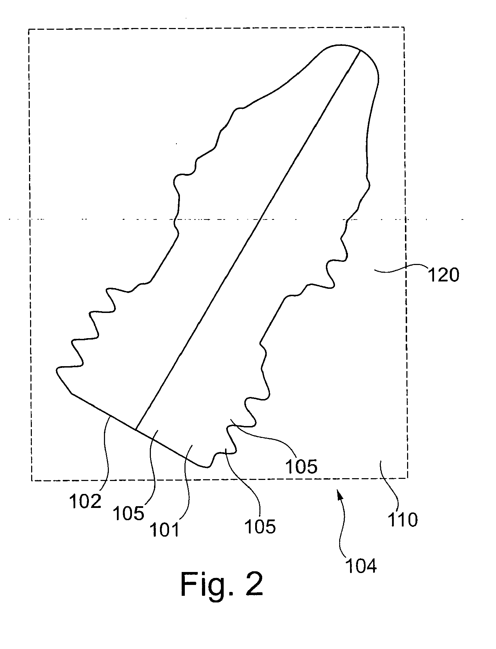

Fig. 2 illustrates determining a contour of a product in a package. After obtaining an image of the product 101 in a package 110, an outer contour 102 may be determined which separates the product 101 from the environment 120 of the product 101. Thus, the product 101 can be determined within the package 110. The determination of the outer contour 102 of the product 101 can be obtained based on a greyscale image 104 having a plurality of greyscales 105. The process of determining a contour is also referred to as segmentation of the product or in general the object.

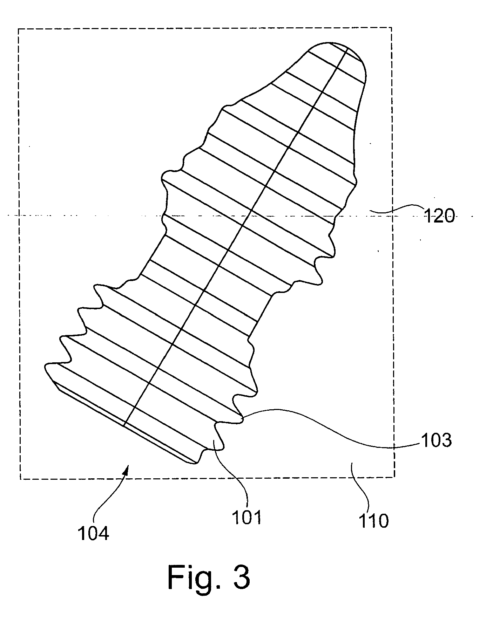

Fig. 3 illustrates the determination of a geometry of the product. The product 101 in the package 110 will be separated and segmented with respect to the environment 120 of the product. Based on the greyscale image 104, the geometry 103 of the product will be determined, which can be supported, for example, by further contour lines representing the geometry of the product.

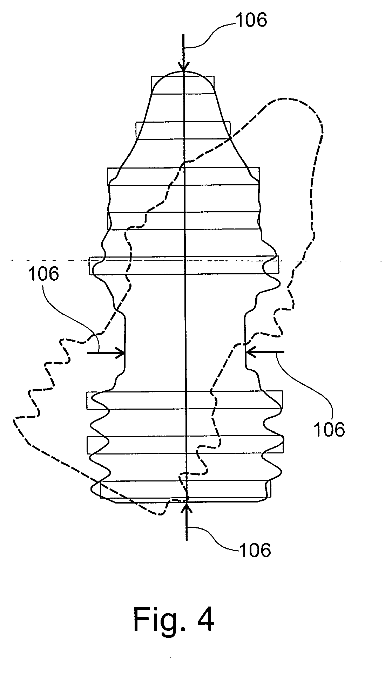

Fig. 4 illustrates the determination of the parameters of the product 101. It should be noted that the determined contour can be rotated with respect to the image in order to obtain a co-ordinate system transformation, which allows an improved and faster determination of the parameters 106. Those parameters may be, for example, the length, the diameter, the material, several distances and further parameters, and are not limited to the aforementioned parameters.

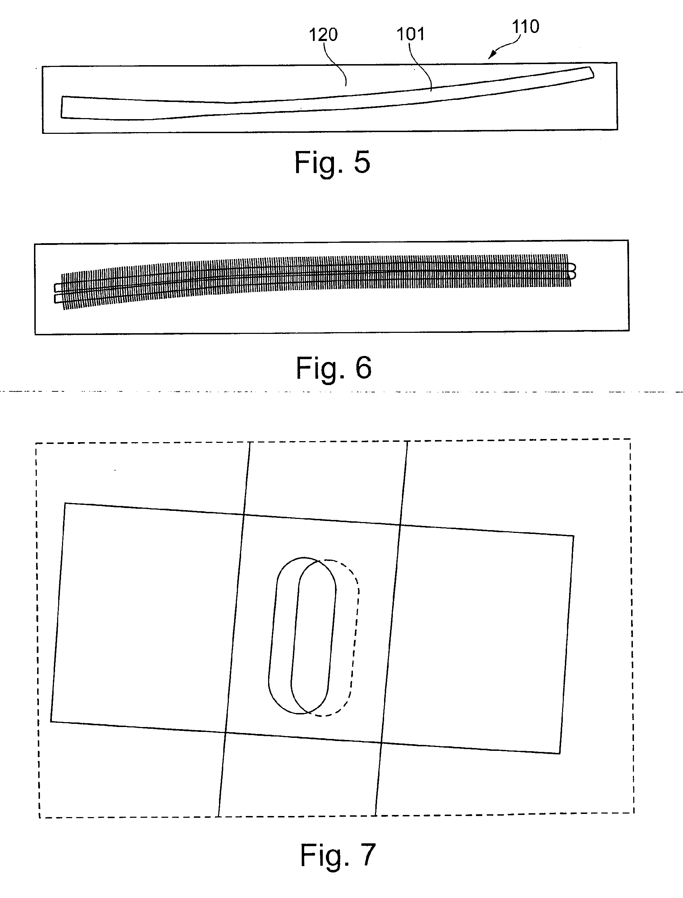

Fig. 5 illustrates an object 101 within a package 110, wherein the image is segmented or separated in the object as such 101 and the environment 120 of the object.

Fig. 6 illustrates a kind of line scanning of the product or more exactly the image of the product. The line scanning allows a sufficient resolution of the image, which again allows a more detailed analysis of the object.

Fig. 7 illustrates the determination of a parameter with respect to a through-hole of the object. In the particular embodiments of Figs. 5, 6 and 7, the object in the package is a bone nail. In particular, with respect to medical or surgical products, it is very important to maintain the reliability of the labelling and the classification of the products, since serious damages and injury of a patient will occur if not considering the required security measurements. In particular, it may be not visible also for a skilled person which particular properties are provided with a particular product. Thus, it is, for example, possible to eliminate human errors in evaluating products and also eliminating human errors in identifying a particular product. For example, a plurality of different bone nails are provided which have, for example, different bores, different bending and different geometries, which are important for a fitted implementation to the human body. Some of the geometries can not be distinguished also by a skilled person at first sight, so that the automated process also provides for a secure and fast determination of the product, identification of the product and also labelling of the product.

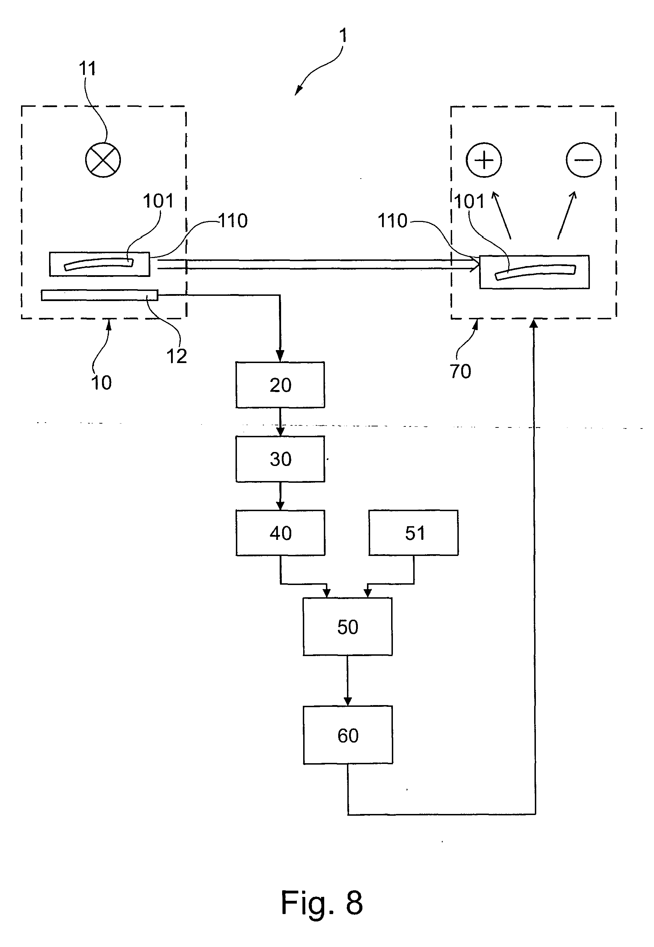

Fig. 8 illustrates an exemplary embodiment of the inventive device. The device 1 comprises an imaging device 10, which may be, for example, and X-ray imaging device with an X-ray source 11. The product 101 within the package 110 is provided in the examination area so that by the exposure apparatus 11 the product 101 in the package 110 can be imaged on an imaging device in form of an X-ray detector 12. It should be noted that also other frequency ranges may be used for an imaging process, and that a skilled person will conceivably adapt the source 11 and the detector 12. The detector 12 or in general the imaging device 10 inputs the image into the contour determining device 20 for determining an outer contour of the product based on the imaging of the product in a package. Afterwards, the result will be provided to a geometry determining device 30, which is adapted for determining the geometry of the product based on the outer contour of the product. A parameter determining device 40 will determine parameters of the product based on the determined geometry of the product, i.e. based on the output of the geometry determining device 30. The comparing device 50 will, for example, compare the parameters of the product with, for example, the entries of a look-up table 51 and provide the result to an evaluating device 60 for evaluating whether the product is a conform product or a non-conform product. It should be noted that based on the result whether the product is a conform product or a non-conform product, a sorting device 70 may be provided which sorts the imaged and evaluated products into a good product, i.e. a conform product denoted with a plus or into a bad product, i.e. a non conform product denoted with a minus.

It should be noted that the present invention may not only be applied to medical or surgical products, but may also be applied to any other packed product, which should be identified with respect to parameters or with respect to labelling.

It should be noted that the term 'comprising' does not exclude other elements or steps and that 'a' or 'an' does not exclude a plurality. Also elements described in association with the different embodiments may be combined.

It should be noted that the reference signs in the claims shall not be construed as limiting the scope of the claims.

## Claims

1. Method for identification of a product (101) in a package (110), the method comprising:
imaging the product (S10);
determining an outer contour of the product (S20) based on the imaging of the product in a package;
determining a geometry of the product (S30) based on the outer contour (102) of the product;
determining parameters of the product (S40) based on the determined geometry (103) of the product;
comparing determined parameters of the product (S50) with respective target parameters of the product;
evaluating the product (S60) as a conform product or as a non-conform product.

2. Method according to claim 1, wherein imaging of the product is an X-ray imaging of the product.

3. Method according to claim 1 or 2, wherein determining the outer contour of the product comprises determining a gray scale image (104) of the product (S21) based on the imaging of the product and separating (S21) the product from an environment (120) of the product by applying a predetermined threshold to the gray scale image.

4. Method according to anyone of claims 1 to 3, wherein determining a geometry of the product comprises determining a gray scale image (104) of the product (S31) based on the outer contour of the product and determining the geometry of the product (S32) based on a plurality of gray scales (105) of the gray scale image.

5. Method according to anyone of claims 1 to 4, wherein determining parameters of the product comprises determining a parameter out of a group, the group consisting of dimension, material, shape, radius, angle, diameter, hole diameters, threads.

6. Method according to anyone of claims 1 to 5, wherein comparing determined parameters of the product with respective target parameters of the product comprises comparing the determined parameters with a look-up table (S51).

7. Method according to anyone of claims 1 to 6, further comprising reading a label on the package (S91), identifying a label content (S92), allocating to the label content corresponding target parameters (S93) and comparing the determined parameters of the product with allocated target parameters (S94).

8. Method according to anyone of claims 1 to 7, further comprising sorting the product (S70) based on evaluating the product as a conform product or as a non-conform product.

9. Method according to anyone of claims 1 to 8, further comprising providing a tag (130) to the package (S81) based on evaluating the product as a conform product or as a non-conform product and sealing the package together with the tag (S82).

10. Device for identification of a product (101) in a package (110), the device (1) comprising:
imaging device (10) being adapted for imaging the product;
contour determining device (20) being adapted for determining an outer contour (102) of the product based on the imaging of the product in a package;
geometry determining device (30) being adapted for determining a geometry (103) of the product based on the outer contour of the product;
parameter determining device (40) being adapted for determining parameters of the product based on the determined geometry of the product;
comparing device (50) being adapted for comparing determined parameters of the product with respective target parameters of the product;
evaluating device (60) being adapted for evaluating the product as a conform product or as a non-conform product.

11. Device according to claim 10, wherein the imaging device is an X-ray line scanner.

## Amended claims

### Amended claims in accordance with Rule 137(2) EPC.

**1.** Method for identification of a medical product (101) in a package (110), the method comprising:
imaging the product (S10);
determining an outer contour of the product (S20) based on the imaging of the product in a package, wherein determining the outer contour of the product comprises determining a gray scale image (104) of the product (S21) based on the imaging of the product and separating (S21) the product from an environment (120) of the product by applying a predetermined threshold to the gray scale image;
determining a geometry of the product (S30) based on the outer contour (102) of the product;
determining parameters of the product (S40) based on the determined geometry (103) of the product;
comparing determined parameters of the product (S50) with respective target parameters of the product, wherein comparing determined parameters of the product with respective target parameters of the product comprises comparing the determined parameters with a look-up table (S51);
reading a label on the package (S91), identifying a label content (S92), allocating to the label content corresponding target parameters (S93) and comparing the determined parameters of the product with allocated target parameters (S94)
evaluating the product (S60) as a conform product or as a non-conform product.

**2.** Method according to claim 1, wherein imaging of the product is an X-ray imaging of the product.

**3.** Method according to anyone of claims 1 to 2, wherein determining a geometry of the product comprises determining a gray scale image (104) of the product (S31) based on the outer contour of the product and determining the geometry of the product (S32) based on a plurality of gray scales (105) of the gray scale image.

**4.** Method according to anyone of claims 1 to 3, wherein determining parameters of the product comprises determining a parameter out of a group, the group consisting of dimension, material, shape, radius, angle, diameter, hole diameters, threads.

**5.** Method according to anyone of claims 1 to 4, further comprising sorting the product (S70) based on evaluating the product as a conform product or as a non-conform product.

**6.** Method according to anyone of claims 1 to 5, further comprising providing a tag (130) to the package (S81) based on evaluating the product as a conform product or as a non-conform product and sealing the package together with the tag (S82).

**7.** Device for identification of a medical product (101) in a package (110), the device (1) comprising:
imaging device (10) being adapted for imaging the product;
contour determining device (20) being adapted for determining an outer contour (102) of the product based on the imaging of the product in a package wherein determining the outer contour of the product comprises determining a gray scale image (104) of the product based on the imaging of the product and separating the product from an environment (120) of the product by applying a predetermined threshold to the gray scale image;
geometry determining device (30) being adapted for determining a geometry (103) of the product based on the outer contour of the product;
parameter determining device (40) being adapted for determining parameters of the product based on the determined geometry of the product;
comparing device (50) being adapted for comparing determined parameters of the product with respective target parameters of the product wherein comparing determined parameters of the product with respective target parameters of the product comprises comparing the determined parameters with a look-up table;
reading device being adapted for reading a label on the package, identifying a label content, allocating to the label content corresponding target parameters and comparing the determined parameters of the product with allocated target parameters;
evaluating device (60) being adapted for evaluating the product as a conform product or as a non-conform product.

**8.** Device according to claim 70, wherein the imaging device is an X-ray line scanner.
